# EUROPEAN PATENT APPLICATION

(11) **EP 4 703 911 A1**
(43) Date of publication of application: **04.03.2026**
(21) Application number: 24197542.4
(22) Date of filing: 30.08.2024
(51) Int. Cl.: G06F 16/55

(54) **MEDICAL IMAGE CURATION**

(71) Applicant: Koninklijke Philips N.V., 5656 AG Eindhoven (NL)
(72) Inventor: AWASTHI, Manish, Eindhoven (NL); SUBASH CHANDRAN, Dheepak, Eindhoven (NL); AIRSANG, Urmila, 5656AG Eindhoven (NL); KOTHAWALA, Aliarshad Aameer, Eindhoven (NL); VAYYETI, Anudeep, Eindhoven (NL); NELLUR PRAKASH, Sindhu Priyadarshini, Eindhoven (NL); VELUMURGAN, Mylavan, Eindhoven (NL); SHETTIGAR, Srikanth, Eindhoven (NL); FIRTION, Celine, Eindhoven (NL); NANJUNDEGOWDA, Navya, Eindhoven (NL); VAJINEPALLI, Pallavi, Eindhoven (NL)
(74) Representative: Philips Intellectual Property & Standards

(57) **Abstract**

The present disclosure provides concepts for curating medical images of an anatomical structure acquired during an examination. Curating the medical images includes obtaining a user preference indicating desired anatomical sub-structures in a target view plane, generating a segmentation score for each medical image depicting the target view plane, and identifying primary medical images based on the segmentation scores. The segmentation score indicates a confidence of the desired anatomical sub-structures being present in the respective medical image. The invention enables customized curation of medical images based on user-specified anatomical features, improving efficiency and relevance of selected medical images from an examination, such that subsequent medical image analysis may be more effective and efficient.

## Description

### FIELD OF INVENTION

The present invention relates to medical image curation systems and methods, and more particularly to systems and methods for automatically identifying and selecting medical images of anatomical structures.

### BACKGROUND

Fetal heart screening is a critical component of prenatal care, performed in the early second trimester to identify congenital heart diseases (CHDs). CHDs are the most common type of birth defect, affecting approximately one percent of live births. While CHDs may be asymptomatic during fetal development, they can lead to significant morbidity and mortality after birth. Early diagnosis of CHDs is crucial for improving outcomes and expanding therapeutic options, including emerging in-utero treatments for specific lesions.

Despite the potential benefits of early detection, the fetal diagnosis rate for CHDs is low (typically between 30-50%), even in developed nations with universal fetal ultrasound screening. This diagnosis gap is primarily attributed to inadequate expertise in acquiring and interpreting fetal cardiac images. The small size and rapid beating of the fetal heart, combined with the subtle and varied nature of cardiac abnormalities, present significant challenges for clinicians in capturing and interpreting fetal cardiac images.

Current guidelines recommend acquiring multiple standard view planes of the fetal heart to provide comprehensive information about its structure and function. However, the acquisition of these view planes and the identification of relevant anatomical structures can be challenging, particularly for less experienced clinicians. Of course, this problem also exists for imaging and interpreting of anatomical structures other than the fetal heart (e.g., adult heart, brain, liver, etc.).

Accordingly, there exists a need for an improved means for curation of medical images of the anatomical structures. Specifically, the identification of appropriate images of the anatomical structure may help to bridge the gap between the theoretical detection rate of diseases, and the actual diagnosis rate in clinical practice.

### SUMMARY OF INVENTION

The invention is defined by the claims.

According to an aspect of the present disclosure, a method for curating a plurality of medical images of an anatomical structure acquired during an examination of a subject is provided. The method includes obtaining a user preference indicating one or more desired anatomical sub-structures in a target view plane of the anatomical structure. The method further includes generating, for each of the medical images depicting the target view plane, a segmentation score indicating a confidence of the one or more anatomical sub-structures indicated by the user preference being present in the respective medical image. The method also includes identifying one or more primary medical images of the target view plane amongst the plurality of medical images based at least in part on the segmentation scores.

The present invention provides a novel method for curating medical images of anatomical structures, particularly ultrasound images of a fetal heart, by incorporating user preferences and real-time analysis. This approach addresses the problems associated with existing medical image curation methods, where selected medical images are often of low quality, and lacking features that are preferred by the clinician. For example, expertise in acquiring and interpreting cardiac images (and particularly fetal cardiac images) is often lacking, leading to low diagnosis rates for congenital heart diseases.

In particular, the user provides preferences indicating anatomical sub-structures that they wish to be present in the selected medical image (i.e., identified primary medical image) of a view plane. Each medical image of the view plane is therefore assessed to provide a segmentation score indicating a likelihood that the indicated anatomical sub-structures are present. One or more medical images are therefore identified based on the segmentation scores, thus providing medical images that are most likely to depict all desired anatomical sub-structures.

The user preference may further comprise a weighting factor for each of the one or more desired anatomical sub-structures that the user wishes to be present in the selected medical image. In this way, the user can indicate that the presence of certain anatomical sub-structures is regarded as being more important that the presence of others. In such cases, the segmentation score can then be generated taking into account the weighting factors. That is, the segmentation score may be a weighed combination, wherein the likelihood that each indicated anatomical sub-structure is present is weighed by the weighting factor for said indicated anatomical sub-structure.

The invention leverages techniques to automatically classify anatomical structures in medical images captured in real-time during examinations. A key innovation is the incorporation of user-configurable preferences for specific anatomical sub-structures within each view plane. This allows the system to adapt to individual clinician preferences and practices while still adhering to established guidelines. By accommodating clinician preferences, diagnosis rates may be improved.

By combining automated image analysis with user-defined preferences, this invention has the potential to streamline workflow, improve diagnostic accuracy, and ultimately enhance the detection rate of anomalies of the anatomical structure of a subject. The method's flexibility and adaptability make it a valuable tool for both experienced clinicians and novice users, potentially bridging the gap between the theoretical and actual diagnosis rates using medical images.

In some embodiments, the method may include identifying temporal clusters of the plurality of medical images comprising consecutive medical images depicting the target view plane. This feature allows improved efficiency in image analysis, with consecutive medical images likely to depict the target view plane.

The method may further include generating, for each identified temporal cluster, a cluster score indicating a quality of the images in the respective temporal cluster. This quality assessment of grouped images is efficient, as consecutive images are likely to be of similar quality.

The identification of the primary medical images of the target view plane may be further based on the cluster scores, allowing for selection of high-quality images. Indeed, by taking into account both segmentation score and cluster scores, images that both comprise the desired anatomical sub-structures and depict such sub-structures with satisfactory quality may be identified.

More specifically, the identification of the primary medical images may comprise identifying a primary medical image cluster amongst the temporal clusters based at least in part on the segmentation scores and the cluster scores. This may facilitate selection of the best group of related images. Indeed, it may be desirable to select and bookmark clusters of images that are likely to contain the desired sub-structures whilst being of good quality.

In some embodiments, the method may also include, for each of a plurality of different view planes of the anatomical structure: obtaining a user preference indicating one or more desired anatomical sub-structures in the respective view plane of the anatomical structure; generating, for each of the medical images depicting the respective view plane, a segmentation score indicating a confidence of the one or more anatomical sub-structures indicated by the associated user preference being present in the respective medical image; and identifying one or more primary medical images of the respective view plane amongst the plurality of medical images based at least in part on the segmentation scores.

This feature allows for comprehensive analysis across multiple view planes. Indeed, it is often the case that multiple view planes of an anatomical structure are desired (e.g., to satisfy guidelines). For each of these target view planes, there may be different anatomical sub-structures that would be beneficial to be present. Thus, this feature enables the identification of medical images that provide a comprehensive view of the whole anatomical structure.

The method may include classifying each of the plurality of medical images, each class corresponding to one of the plurality of view planes of the anatomical structure. The method may further include generating, for each of the medical images, a classification score indicating a confidence of the respective medical image belonging to the associated class, providing a measure of classification accuracy. The identification of the primary medical images for each of a plurality of view planes may be further based on the classification scores.

Thus, the above features enable the automatic categorization of images into classes of the appropriate view plane for subsequent analysis, which may then be used for identification of the primary medical images. Indeed, it is important that the selected image depicts the intended target view plane.

The method may include generating a bookmark of medical images comprising the one or more primary medical images of each of the plurality of different view planes of the anatomical structure. This may facilitate easy access to a comprehensive catalogue of the medical images, the catalog comprising images of each of the target view planes with the highest likelihood of depicting all desired anatomical sub-structures.

The generation of the segmentation score may comprise: generating, for each of the anatomical sub-structures, a confidence score indicating a likelihood of the anatomical sub-structure being present in the medical image; and generating the segmentation score based on the confidence scores.

The method may include receiving the plurality of medical images from an imaging system in real-time. This therefore enables immediate analysis during examination, so that the clinician may take appropriate action should additional or alternative images be required.

In specific cases, the anatomical structure may be a heart, and optionally the target view plane may be a four chamber cardiac view, a left ventricular outflow tract view, a right ventricular outflow tract view, a three vessels and trachea view, or a three vessel view. Indeed, the invention may be particularly useful for cardiac analysis.

According to another aspect of the present disclosure, a computer program comprising computer program code means adapted, when said computer program is run on a computer, to implement the method described above is provided.

According to another aspect of the present disclosure, a system for curating a plurality of medical images of an anatomical structure acquired during an examination of a subject is provided. The system includes an interface configured to obtain a user preference indicating one or more desired anatomical sub-structures in a target view plane of the anatomical structure. The system also includes a processor configured to: generate, for each of the medical images depicting the target view plane, a segmentation score indicating a confidence of the one or more anatomical sub-structures indicated by the user preference being present in the respective medical image; and identify one or more primary medical images of the target view plane amongst the plurality of medical images based at least in part on the segmentation scores.

These and other aspects of the invention will be apparent from and elucidated with reference to the embodiment(s) described hereinafter.

### BRIEF DESCRIPTION OF FIGURES

For a better understanding of the invention, and to show more clearly how it may be carried into effect, reference will now be made, by way of example only, to the accompanying drawings, in which:
FIG. 1 illustrates a flowchart of a method for curating medical images of an anatomical structure, according to aspects of the present disclosure;
FIG. 2 illustrates a flowchart of a method for curating medical images, according to an embodiment;
FIG. 3 illustrates a block diagram of a system for curating medical images, in accordance with example embodiments;
FIG. 4 is a simplified block diagram of a computer within which one or more parts of an embodiment may be employed.

### DETAILED DESCRIPTION

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to provide an advantage.

The present disclosure provides concepts for curating medical images of an anatomical structure acquired during an examination. Curating the medical images includes obtaining a user preference indicating desired anatomical sub-structures in a target view plane, generating a segmentation score for each medical image depicting the target view plane, and identifying primary medical images based on the segmentation scores. The segmentation score indicates a confidence of the desired anatomical sub-structures being present in the respective medical image. The invention enables customized curation of medical images based on user-specified anatomical features, improving efficiency and relevance of selected medical images from an examination, such that subsequent medical image analysis may be more effective and efficient.

In an exemplary embodiment, the invention generates a composite score for each image based on view plane classification, bookmark classification, and segmentation of desired sub-structures. This scoring mechanism enables the automatic identification and selection of high-quality, clinically relevant images that meet the specific criteria set by the user. By prioritizing images containing the features deemed most important by the clinician, the invention can significantly improve the efficiency and accuracy of analysis of images generated during a screening/examination.

Furthermore, the systems and methods may provide real-time feedback and guidance to users, potentially improving the quality of acquired images and helping less experienced users to systematically capture all relevant standard planes. This feature could be particularly valuable in addressing the uneven expertise in imaging that contributes to a diagnosis gap.

By combining automated image analysis with user-defined preferences, these systems and methods have the potential to streamline workflow, improve diagnostic accuracy, and ultimately enhance the detection rate of congenital heart diseases in prenatal screenings. The system's flexibility and adaptability make it a valuable tool for both experienced clinicians and novice users, potentially bridging the gap between theoretical and actual diagnosis rates.

In specific embodiments, the present disclosure provides systems and methods for enhancing the process of fetal heart screening through the automated analysis of medical images. These systems and methods leverage techniques to automatically classify and segment fetal heart view planes in real-time during ultrasound examinations.

A key aspect of these systems and methods is the incorporation of user-configurable preferences for specific anatomical sub-structures within each view plane. This allows the system to adapt to individual clinician preferences and practices while still adhering to established guidelines. For example, for a four chamber cardiac view plane, it is usually preferred that the image includes symmetrical and well visible chambers in systole phase. Nevertheless, for some users, pulmonary veins are the most important feature to be present in the image. Similarly, for a three vessel view it is usually preferred that the image depicts the three main vessels. However, some users also wish for the connecting ductus arteriosus to also be depicted.

The invention thus allows for these preferences to be taken into account when identification of primary images amongst the medical images acquired during the examination. Indeed, in existing systems, it is acknowledged that it is not possible to satisfy the preferences of all users. Such systems are typically trained to be fixed on specific inputs, and therefore cannot change according to user preference. In contrast, the proposed solution enables the selection of user preferences. In some cases, the user preference may be provided in the form of a configuration file in which the user provides their preferences before the invention provides the automatic selection of primary medical images amongst the full stream of medical images.

In other words, existing solutions provide medical images to a classifier which is trained to detect standard view planes amongst the medical images. A score is provided for each medical image, which takes into account the confidence that the medical image depicts a certain view plane, and also the quality of the medical image. Medical images with the highest associated score for each target view plane are then compiled for interpretation by a clinician.

In the case of the present invention, a segmentation score is generated for each medical images, which indicates the likelihood that the medical image depicts anatomical sub-structures that are indicated by the user. Accordingly, the invention ensures that the selected medical images for further analysis by the clinician are the most appropriate according to user preferences. Of course, in some cases the invention will also consider the confidence that the medical image depicts a certain view plane, and the quality of the medical image, and therefore may select the medical images based on each of these factors.

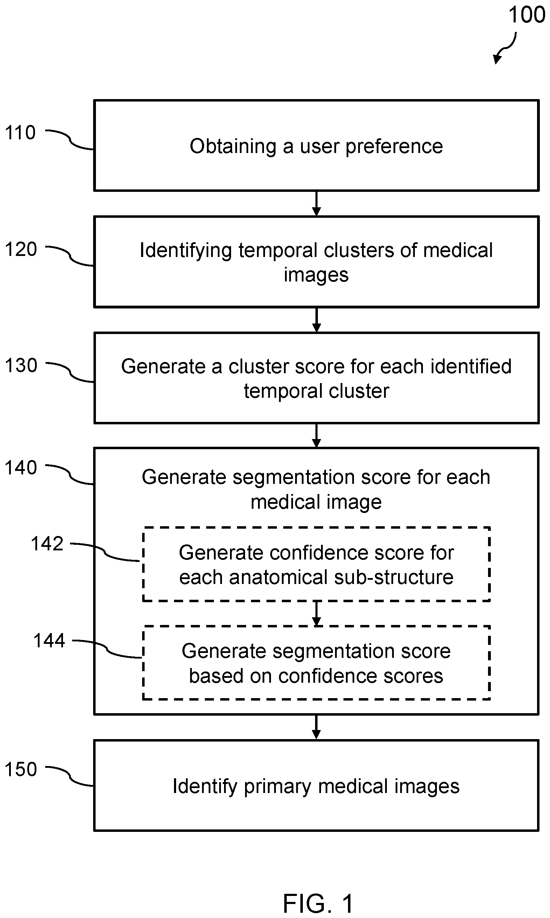

Referring to FIG. 1, a method 100 for curating medical images of an anatomical structure is illustrated. To be clear, the medical images may be of any anatomical structure. The medical images are acquired during an examination of a subject, such as an ultrasound scan.

Furthermore, in some cases, the plurality of medical images may be acquired in real time. That is, steps 120-150 of method 100 may be performed during the examination of the subject, such that primary medical images are identified during the examination of the subject. Thus, in some aspects, the method may also involve receiving the plurality of medical images from an imaging system in real-time. This real-time processing allows the system to provide immediate feedback to the user, potentially improving the quality of the acquired images and helping the user to systematically capture all relevant standard view planes.

In step 110, a user preference is obtained. The user preference indicates one or more desired anatomical sub-structures in a target view plane of the anatomical structure. The user preference may be input by a clinician or other user and may vary based on the specific clinical context or individual preferences. In some cases, the user preference may be stored and retrieved from a user profile or other data storage system. In other cases, the user may be required to provide a configuration file before the acquisition of medical images begins.

In (optional) step 120 temporal clusters of the plurality of medical images are identified. These temporal clusters comprise consecutive medical images depicting a same target view plane. That is, sequences of medical images that each present a particular view plane are identified amongst the plurality of medical images. The temporal clusters may comprise at least five medical images, for example.

The temporal clusters may be identified based on the sequence and timing of the acquired medical images. In some cases, the temporal clusters may be identified using clustering algorithms or other data analysis techniques.

Once the temporal clusters are identified 120, the method 100 may then involve generating 130 a cluster score for each identified temporal cluster. The cluster score indicates the quality of the images in the respective temporal cluster. The cluster score may be generated based on various factors, such as the consistency of the images within the cluster, the extent of noise and other artifacts in the images, and other relevant factors. In some cases, the cluster score may be a composite score that takes into account multiple factors. The cluster score may be used to prioritize certain clusters over others and to guide the selection of the primary medical images based on quality of the image.

In step 140, a segmentation score for each of the medical images depicting the target view plane is generated. The segmentation score indicates a confidence of the one or more anatomical sub-structures indicated by the user preference being present/observable in the respective medical image of the target view plane. In other words, the segmentation score may be a composite score that takes into account multiple factors, such as the presence, and visibility of the desired sub-structures, and other relevant factors related to the desired sub-structures. The segmentation score may be generated using a deep learning model or other machine learning techniques.

The generation 140 of the segmentation score may involve a two-step process. First, for each of the anatomical sub-structures, the system may generate 142 a confidence score indicating a likelihood of the anatomical sub-structure being present in the medical image. This confidence score may be generated using a deep learning model or other machine learning techniques. The confidence score may take into account the visibility and clarity of the sub-structure in the image, for example. Second, the system may generate 144 the segmentation score of each medical image based on the respective confidence scores. The segmentation score may be a composite score that takes into account the confidence scores of all the desired sub-structures.

In step 150, one or more primary medical images of the target view plane amongst the plurality of medical images are identified. This identification is based at least in part on the segmentation scores. The primary medical images thus are those that best meet the user's preferences and provide the most clinically relevant information. In some cases, the primary medical images may be automatically selected by the system based on the segmentation scores. In other cases, the system may present a ranked list of potential primary medical images to the user for manual selection.

In some cases, the identification 150 of the primary medical images may be based on both the segmentation scores and the cluster scores. This dual scoring system allows for a more nuanced and accurate selection of primary medical images. For example, an image with a high segmentation score (indicating a high confidence of the presence of the desired sub-structures in the medical image) but a low cluster score (indicating a low quality of the medical image belong to the temporal cluster) may not be selected as a primary medical image. Conversely, an image with a high cluster score but a low segmentation score may also not be selected. In this way, the system may prioritize images that both meet the user's preferences and are of high quality.

In some aspects, the identification 150 of the primary medical images may involve identifying a primary medical image cluster amongst the temporal clusters. This identification may be based at least in part on the segmentation scores and the cluster scores. The primary medical image cluster is the cluster that best meets the user's preferences and provides the most clinically relevant information. The identification of a primary medical image cluster can help to streamline the image review process by grouping together high-quality, relevant images.

It should be noted that the method 100 may also be applied to multiple different view planes of the anatomical structure. For each of these view planes, the system may obtain 110 a user preference indicating one or more desired anatomical sub-structures in the respective view plane. The system may then generate 140 a segmentation score for each of the medical images depicting the respective view plane. This score indicates a confidence of the one or more anatomical sub-structures indicated by the associated user preference being present in the respective medical image. The system may then identify 150 one or more primary medical images of the respective view plane amongst the plurality of medical images based at least in part on the segmentation scores. This multi-view plane approach allows the system to provide comprehensive and customized image analysis for a range of different anatomical views.

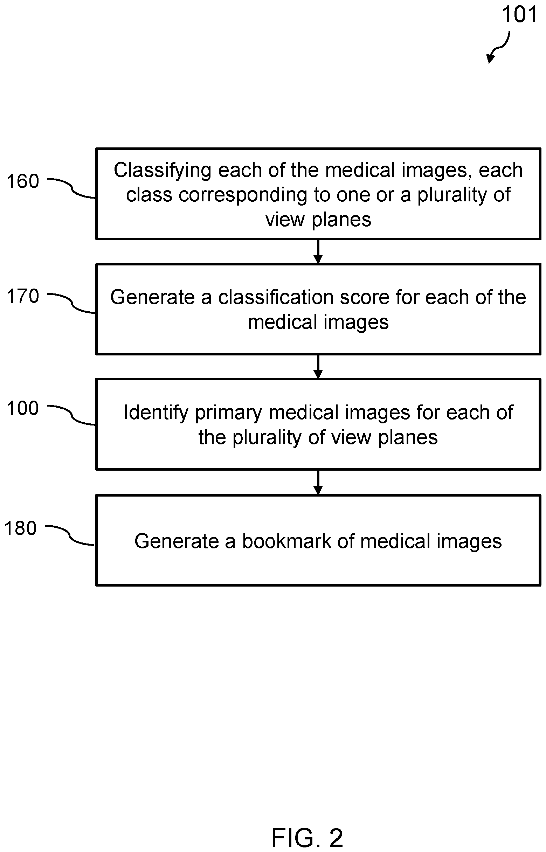

Referring to FIG. 2, a method 101 for curating medical images is illustrated, which may be used to identify primary medical images for a plurality of view planes.

In step 160, each of the medical images are classified, where each class corresponds to one or a plurality of view planes of the anatomical structure. This classification process may be performed using a deep learning model or other machine learning techniques. The classification process may take into account various factors, such as the orientation, angle, and visibility of the anatomical structure in the image.

In other words, a view plane depicted in each of the medical images is identified, and the medical images then classified accordingly.

In step 170, a classification score is generated for each of the medical images. The classification score indicates a confidence in the respective medical image belonging to the associated class. That is, medical images that are very likely to depict the view plane of the associated class is given a high confidence score.

Then, a method for identifying primary medical images for each of the plurality of view planes is performed. This identification may comprise some or all of the steps of method 100. That is, a segmentation score (and optionally a cluster score) is generated for each of the medical images. Then, primary medical images for each of the plurality of view planes can be identified based on the classification scores and segmentation scores (and cluster scores, if generated). The primary medical images are thus those that best meet the user's preferences, and best match the respective target view plane (and are of high quality, if the cluster score is generated). In some cases, the primary medical images may be automatically selected by the system based on the scores. In other cases, the system may present a ranked list of potential primary medical images to the user for manual selection.

In some cases, the method may also involve adjusting the weights associated with the classification score, segmentation score, and cluster score based on user input. This allows the system to adapt to individual user preferences and practices, potentially improving the relevance and usefulness of the curated images.

In step 180, a bookmark of medical images is generated. The bookmark comprises the primary medical images of each of the plurality of view planes of the anatomical structure. The bookmark therefore provides a curated set of images that are organized according to their view plane and quality. The bookmark may be stored in a data storage system for later retrieval and review. In some cases, the bookmark may be presented to the user in a graphical user interface, allowing the user to easily navigate through the curated images.

It will be appreciated that the steps represented in FIG. 1 and FIG. 2 may be performed in different orders. For example, step 120 and 130 may be performed before steps 110 and 140. Accordingly, the invention is not intended to be limited by the depicted ordering of the steps presented in these Figures.

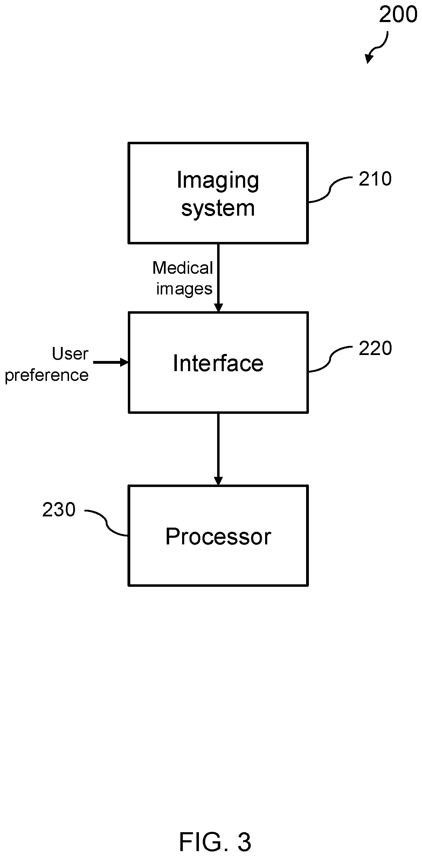

Referring to FIG. 3, a system 200 for curating medical images of an anatomical structure is illustrated. The system 200 comprises an imaging system 210, an interface 220, and a processor 230. The imaging system 210 generates medical images of an anatomical structure during an examination of a subject. In some cases, the imaging system 210 may be an ultrasound system used for fetal heart screening. However, in other cases, the imaging system 210 may be any suitable medical imaging system capable of generating images of an anatomical structure, such as a computed tomography (CT) scanner, a magnetic resonance imaging (MRI) scanner, or a positron emission tomography (PET) scanner.

The interface 220 is configured to obtain a user preference indicating one or more desired anatomical sub-structures in a target view plane of the anatomical structure. The user preference may be inputted by a clinician or other user and may vary based on the specific clinical context or individual preferences. In some cases, the user preference may be stored and retrieved from a user profile or other data storage system. The interface 220 may also receive the plurality of medical images from the imaging system 210 in real-time. This real-time processing allows the system 200 to provide immediate feedback to the user, potentially improving the quality of the acquired images and helping the user to systematically capture all relevant standard view planes.

The processor 230 is configured to generate, for each of the medical images depicting the target view plane, a segmentation score indicating a confidence of the one or more anatomical sub-structures indicated by the user preference being present in the respective medical image. The processor 230 may use a deep learning model or other machine learning techniques to generate the segmentation score. In some cases, the segmentation score may be a composite score that takes into account multiple factors, such as the presence and visibility of the desired sub-structures, the quality of the image, and other relevant factors.

The processor 230 is also configured to identify one or more primary medical images of the target view plane amongst the plurality of medical images based at least in part on the segmentation scores. The primary medical images are those that best meet the user's preferences and provide the most clinically relevant information. In some cases, the primary medical images may be automatically selected by the processor 230 based on the segmentation scores. In other cases, the processor 230 may present a ranked list of potential primary medical images to the user for manual selection.

In some cases, the system 200 may also include a display (not shown) for presenting the medical images, the segmentation scores, and other relevant information to the user. The display may be any suitable type of display device, such as a monitor, a touchscreen, or a projector. The display may be communicatively coupled to the processor 230, allowing the processor 230 to control the display and present the relevant information to the user.

In some aspects, the processor 230 may include a detection module that utilizes a detection deep neural network. This detection module may be configured to receive an input stream of 2D ultrasound images from the imaging system 210 via the interface 220. The detection module may be trained to identify all possible structures and orientations of the anatomical structure in each medical image, with all other anatomical structures in the medical image detected as background. This allows the detection module to accurately and efficiently localize the anatomical structure of interest within each image.

In some cases, the processor 230 may also include a guidance module. The guidance module may take input from the detection module, receiving localized images once the anatomical structure has been detected. The guidance module may be configured to classify the medical images into classes based on the view plane depicted by the medical image. Each class may thus correspond to clinically acceptable views for reporting.

The processor 230 may also include a configurable bookmarking module. The configurable bookmarking module may utilize a segmentation model for real-time segmentation of anatomical sub-structures. The configurable bookmarking module may be configured to generate a segmentation score for each medical image, indicating the confidence of the presence of each sub-structure in the image. The segmentation score may be used in a new scoring mechanism for identification of primary medical images (i.e., may be used, at least in part, to identify primary medical images). For example, an overall score (i.e., a bookmark score) may be generated based on the segmentation score. In one case, each medical image may have an associated overall score, which may comprise a weighted sum of the segmentation score, the cluster score, and the class score as described above.

In some cases, the configurable bookmarking module may allow users to set weights for different anatomies or features in a configuration file before the start of a scan. These weights may be used to adjust the importance of different anatomical sub-structures in the scoring mechanism. For example, a user may assign a higher weight to the presence of the pulmonary veins in a four chamber cardiac view, or the presence of the trachea in a three vessels and trachea view. This user-configurable weighting system may allow the system 200 to adapt to individual user preferences and practices, potentially improving the relevance and usefulness of the curated images.

By way of specific example, the target view plane may be a specific cardiac view, such as a four chamber cardiac view, a left ventricular outflow tract view, a right ventricular outflow tract view, a three vessels and trachea view, or a three vessel view. The user preference obtained by the interface 220 may indicate one or more desired anatomical sub-structures in the target view plane. These sub-structures may include, for example, the chambers of the heart, the valves of the heart, the heart muscle, the pulmonary veins, the aorta, the ductus arteriosus, the trachea, or any other relevant anatomical sub-structures. The user preference may be used to customize the automatic acquisition of view planes according to the user's clinical preferences and practices.

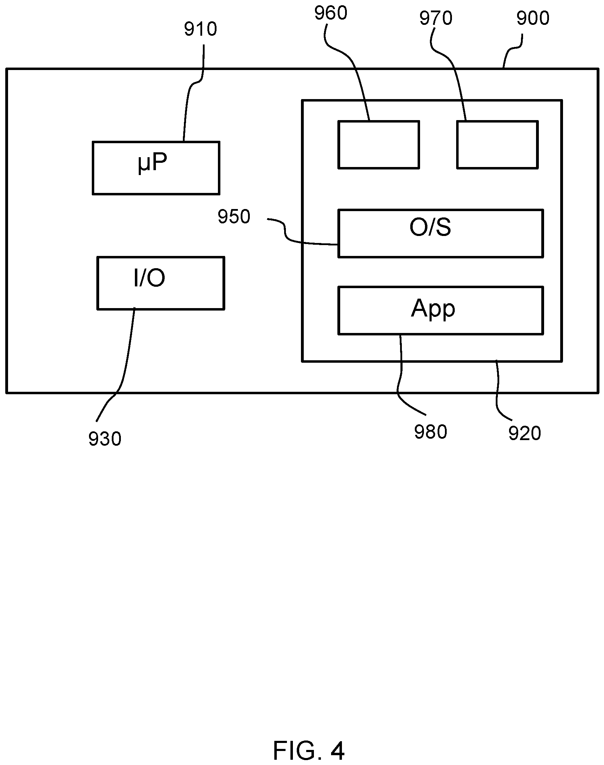

FIG. 4 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc.

Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to provide an advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A method (100) for curating a plurality of medical images of an anatomical structure acquired during an examination of a subject, the method comprising:
obtaining (110) a user preference indicating one or more desired anatomical sub-structures in a target view plane of the anatomical structure;
generating (140), for each of the medical images depicting the target view plane, a segmentation score indicating a confidence of the one or more anatomical sub-structures indicated by the user preference being present in the respective medical image; and
identifying (150) one or more primary medical images of the target view plane amongst the plurality of medical images based at least in part on the segmentation scores.

2. The method of claim 1, further comprising identifying (120) temporal clusters of the plurality of medical images comprising consecutive medical images depicting the target view plane.

3. The method of claim 2, further comprising generating (130), for each identified temporal cluster, a cluster score indicating a quality of the images in the respective temporal cluster.

4. The method of claim 3, wherein identifying (150) the primary medical images of the target view plane is further based on the cluster scores.

5. The method of claim 3 or 4, wherein identifying (150) the primary medical images comprises identifying a primary medical image cluster amongst the temporal clusters based at least in part on the segmentation scores and the cluster scores.

6. The method of any of claims 1-5, further comprising, for each of a plurality of different view planes of the anatomical structure:
obtaining (110) a user preference indicating one or more desired anatomical sub-structures in the respective view plane of the anatomical structure;
generating (140), for each of the medical images depicting the respective view plane, a segmentation score indicating a confidence of the one or more anatomical sub-structures indicated by the associated user preference being present in the respective medical image; and
identifying (150) one or more primary medical images of the respective view plane amongst the plurality of medical images based at least in part on the segmentation scores.

7. The method of claim 6, further comprising classifying (160) each of the plurality of medical images, each class corresponding to one of the plurality of view planes of the anatomical structure.

8. The method of claim 7, further comprising generating (170), for each of the medical images, a classification score indicating a confidence of the respective medical image belonging to the associated class.

9. The method of claim 8, wherein identifying (150) the primary medical images for each of a plurality of view planes is further based on the classification scores.

10. The method of any of claims 6-9, further comprising generating (180) a bookmark of medical images comprising the one or more primary medical images of each of the plurality of different view planes of the anatomical structure.

11. The method of any of claims 1-10, wherein generating (140) the segmentation score comprises:
generating (142), for each of the anatomical sub-structures, a confidence score indicating a likelihood of the anatomical sub-structure being present in the medical image; and
generating (144) the segmentation score based on the confidence scores.

12. The method of any of claims 1-11, further comprising receiving the plurality of medical images from an imaging system in real-time.

13. The method of any of claims 1-12, wherein the anatomical structure is a heart, and optionally wherein the target view plane is a four chamber cardiac view, a left ventricular outflow tract view, a right ventricular outflow tract view, a three vessels and trachea view, or a three vessel view.

14. A computer program comprising computer program code means adapted, when said computer program is run on a computer, to implement the method of any of claims 1-13.

15. A system (200) for curating a plurality of medical images of an anatomical structure acquired during an examination of a subject, the system comprising:
an interface (220) configured to obtain a user preference indicating one or more desired anatomical sub-structures in a target view plane of the anatomical structure;
a processor (230) configured to:
generate, for each of the medical images depicting the target view plane, a segmentation score indicating a confidence of the one or more anatomical sub-structures indicated by the user preference being present in the respective medical image; and
identify one or more primary medical images of the target view plane amongst the plurality of medical images based at least in part on the segmentation scores.
